# EUROPEAN PATENT APPLICATION

(11) **EP 1 985 622 A1**
(43) Date of publication of application: **29.10.2008**
(21) Application number: 07425243.8
(22) Date of filing: 23.04.2007
(51) Int. Cl.: C07J 41/00

(54) **Process for the preparation of tauroursodesoxycholic acid**

(71) Applicant: PRODOTTI CHIMICI E ALIMENTARI SPA, 15067 Basaluzzo (Alessandria) (IT)
(72) Inventor: Parenti, Massimo, 15067 Novi Ligure (Alessandria) (IT)
(74) Representative: Pistolesi, Roberto

(57) **Abstract**

The present invention relates to a novel method for preparing tauroursodeoxycholic acid which comprises a step of selective precipitation of the impurities present in the suspension obtained from the reaction of an aqueous solution of sodium taurinate with an acetonic solution of a mixed anhydride of ursodeoxycholic acid with an alkyl chloroformate.

## Description

The present invention relates to a novel method for preparing tauroursodeoxycholic acid, a substance useful in the treatment of biliary disorders and dysfunction, which method comprises a step of selective precipitation of the impurities present in the suspension obtained from the reaction of an aqueous solution of sodium taurinate with an acetonic solution of a mixed anhydride of ursodeoxycholic acid with an alkyl chloroformate.

### PRIOR ART

Italian patent IT1197330 describes the preparation of a mixed anhydride of tauroursodeoxycholic acid by reaction between ursodeoxycholic acid and an alkyl chloroformate in the presence of a tertiary base, which is reacted with an alkaline aqueous solution of taurine to obtain the sodium salt of tauroursodeoxycholic acid. Subsequent addition of hydrochloric acid and removal of the solvents by vacuum concentration make it possible to obtain a residue containing tauroursodeoxycholic acid. Resuspending the residue with ethanol and subsequent filtration remove the unreacted taurine and sodium chloride, while the tauroursodeoxycholic acid remains in solution. The latter is then precipitated out from the solution by addition of a solvent in which said acid is insoluble. The product is finally dissolved in water and reprecipitated in the solvent in order to reduce the solvent content in the precipitate.

European patent EP400695 describes the preparation of tauroursodeoxycholic acid by means of a purification process of the solution obtained by reaction between taurine and a mixed anhydride of ursodeoxycholic acid in the presence of a tertiary base. In order to avoid the addition of a mineral acid (such as hydrochloric acid) to the sodium salt of tauroursodeoxycholic acid, a procedure which entails various purification steps, the solution arising from the reaction between taurine and the mixed anhydride is passed in series through a first strong cationic exchange resin and through a second weak anionic exchange resin. In this manner, the unreacted tertiary base and the hydrochlorides are removed, while the unreacted taurine and ursodeoxycholic acid are eliminated by known methods. Despite this method not involving the addition of mineral acid to liberate the free tauroursodeoxycholic acid, it does not do without the final purification sequence already described in IT1197330, i.e. concentration to dryness of the percolated solution, resuspension with ethanol, filtration and precipitation with acetone, redissolution in water and reprecipitation in acetone.

A requirement thus remains for a process for producing tauroursodeoxycholic acid which makes it possible to obtain a product in high yield with the lowest possible content of impurities.

### DESCRIPTION OF THE INVENTION

It has surprisingly been found that, by adding a specific number of equivalents of an acid to the alkali or alkaline-earth tauroursodeoxycholate suspension, subsequently leaving the resultant suspension to stand for a defined period, filtering the suspension and adding an organic solvent, it is possible selectively to precipitate the tauroursodeoxycholic acid in particularly pure form.

The present invention accordingly provides a process for preparing tauroursodeoxycholic acid comprising the following steps:
a) obtaining an aqueous suspension of an alkali or alkaline-earth salt of tauroursodeoxycholic acid;
b) adding 0.8-1.4 equivalents of an acid;
c) leaving the resultant suspension to stand for a period of between 10 and 180 minutes;
d) filtering the suspension;
e) adding an organic solvent with subsequent precipitation of the tauroursodeoxycholic acid.

An aqueous suspension of the alkali or alkaline-earth salt of tauroursodeoxycholic acid, preferably the sodium salt, is obtained in the presence of a tertiary base, by reaction between a mixed anhydride of ursodeoxycholic acid with an alkyl chloroformate, preferably ethyl chloroformate, and an alkali or alkaline-earth salt of taurine, preferably a sodium salt. From 0.8 to 1.4 equivalents of an acid are added to said suspension in order to precipitate the taurine and the alkali or alkaline-earth hydrochloride. Preferably from 0.9 to 1.2 equivalents and still more preferably from 1 to 1.1 equivalents of the acid are added. The acid is selected from among hydrochloric acid, nitric acid, acetic acid, sulfuric acid or mixtures thereof, with hydrochloric acid being particularly preferred. The concentration of the acid in water is greater than 30% by weight, with a concentration in water of between 32% and 40% by weight being preferred and a concentration in water of between 32% and 36% by weight being still more preferred. The resultant solution is left to stand for a period of between 10 and 180 minutes, at a temperature of preferably between 15 and 30°C, in order to achieve selective precipitation of the taurine and the alkali or alkaline-earth hydrochloride. In particular, periods of between 15 and 120 minutes are preferred, with periods of between 20 and 60 minutes being still more preferred.

Once the mixture of taurine and alkali or alkaline-earth hydrochloride has been filtered out, the tauroursodeoxycholic acid is precipitated by adding an organic solvent. Said organic solvent preferably has a water content of less than or equal to 1% by weight. The organic solvent is preferably a polar organic solvent, such as acetone, tetrahydrofuran, C₂-C₈ ethers, C₂-C₈ acetates and mixtures thereof. The C₂-C₈ ether is preferably ethyl ether and the C₂-C₈ acetate is preferably ethyl acetate. The particularly preferred solvent is acetone. Alternatively, the organic solvent may be a nonpolar organic solvent, such as chloroform, methylene chloride or mixtures thereof.

The tauroursodeoxycholic acid crystallises with 2 moles of water and many parts per million (ppm) of the organic solvent used for precipitation remain trapped in this water of crystallisation.

An additional purification step is thus carried out by dissolving the resultant tauroursodeoxycholic acid in deionised water. Dissolution is preferably carried out in a maximum period of 60 minutes and at a temperature of below 65°C. In order to avoid product losses, it is necessary to use the least possible quantity of water for dissolution; the aqueous solution is thus heated to a temperature such that all the acid passes into solution. It has accordingly been observed that the best ratio by weight of tauroursodeoxycholic acid:water is between 0.1 and 1, preferably between 0.4 and 0.6, and still more preferably approx. 0.48, relative to the dry weight of tauroursodeoxycholic acid.

When all the tauroursodeoxycholic acid has dissolved, the solution is placed under a vacuum such that the temperature drops sufficiently rapidly with the solvent dissolved in the solution being distilled off.

Wet yield is between 90 and 110% of the ursodeoxycholic acid introduced into the reaction for two operations. After vacuum drying, the yield of dried product is between 68% and 82% of the quantity of ursodeoxycholic acid introduced into the reaction.

The following Examples describe the present invention in greater detail, without there being any intention to restrict the scope thereof.

### EXAMPLE 1

### Preparation of a sodium tauroursodeoxycholate suspension

A solution of sodium taurinate is prepared by introducing 95.6 kg of taurine and 30.9 kg of caustic soda flakes with a minimum content of 97%. 186 kg of demineralised water are added and the suspension is stirred for approx. 30 minutes while maintaining a temperature of approx. 15°C. In this manner, the sodium taurinate solution is obtained.

The mixed anhydride of tauroursodeoxycholic acid is then synthesised starting from 250 kg of ursodeoxycholic acid, 64.9 kg of triethylamine and 69.4 kg of ethyl chloroformate. The ursodeoxycholic acid and 1500 litres of acetone are introduced into a reactor at a temperature of less than 10°C. Under the residual vacuum, the triethylamine is added to the suspension and the temperature is reduced to < 0°. When the temperature of the suspension is < 0°C, the ethyl chloroformate is introduced over approx. 15 minutes. Once addition is complete, the resultant mixed anhydride is stirred for approx. 20 minutes. The triethylamine hydrochloride is then centrifuged and washed with acetone.

The solution of sodium taurinate is introduced into the reactor containing the mixed anhydride.

### Preparation of crude tauroursodeoxycholic acid (TUDCA)

Approx. 30 minutes after the end of hydrolysis, 87 kg of concentrated (33% wt./wt.) hydrochloric acid solution are added. Stirring of the suspension is continued for 60 minutes. Precipitation of the sodium chloride and the taurine takes place. The suspension is centrifuged.

Approx. 2300 litres of acetone are added to the filtered solution. After approx. 60 minutes, the crude TUDCA starts to precipitate out.

Stirring is continued for 12 hours.

The crude TUDCA is separated by centrifugation.

Wet yield approx. 350 kg.

Determination of the chromatographic quality of the crude TUDCA reveals the following results:
impurities <0.3%; taurine <0.5%; ursodeoxycholic acid <0.5%.

### EXAMPLE 2

### Preparation of pure tauroursodeoxycholic acid

1100 litres of demineralised water are introduced into a reactor and are heated, with stirring, up to approx. 80°C. 695 kg of wet crude tauroursodeoxycholic acid, obtained according to Example 1, are introduced into a second reactor and the 1100 litres of water which have previously been heated are introduced therein. The suspension is heated to a temperature of 60°C. At this point, the reactor is slowly evacuated. Once no further acetone is distilled off, distillation is terminated, the vacuum is replaced with nitrogen and the solution is cooled to approx 0°C.

Stirring of the suspension is continued for 64 hours at a temperature of between 0 and 5°C.

The purified TUDCA is then recovered by centrifugation, the product being washed with approx 50 litres of demineralised water for each centrifuge batch.

Wet yield 400 kg.

The wet product is then dried under a vacuum at a maximum temperature of 50°C. The chromatographic quality of the purified TUDCA exhibits the following properties:
taurine <0.2%; ursodeoxycholic acid <0.5%; impurities <0.3% acetone <500 ppm

Content by HPLC is greater than 98.5%.

## Claims

1. A process for preparing tauroursodeoxycholic acid comprising the following steps:
a) obtaining an aqueous suspension of an alkali or alkaline-earth salt of tauroursodeoxycholic acid;
b) adding 0.8-1.4 equivalents of an acid;
c) leaving the resultant suspension to stand for a period of between 10 and 180 minutes;
d) filtering the suspension;
e) adding an organic solvent with subsequent precipitation of the tauroursodeoxycholic acid.

2. A process according to claim 1, **characterised in that** said aqueous suspension of an alkali or alkaline-earth salt of tauroursodeoxycholic acid is obtained by reaction between a mixed anhydride of ursodeoxycholic acid with an alkyl chloroformate and an alkali or alkaline-earth salt of taurine.

3. A process according to claim 1, **characterised in that** 0.9-1.2 equivalents of acid are added in step b).

4. A process according to claim 1, **characterised in that** 1-1.1 equivalents of acid are added in step b).

5. A process according to claim 1, **characterised in that** said acid is selected from among hydrochloric acid, nitric acid, acetic acid, sulfuric acid or mixtures thereof.

6. A process according to claim 1, **characterised in that** said acid has a concentration in water of greater than 30% by weight.

7. A process according to claim 1, **characterised in that** said acid has a concentration in water of between 32% and 40% by weight.

8. A process according to claim 1, **characterised in that** said acid is hydrochloric acid having a concentration in water of between 32% and 36% by weight.

9. A process according to claim 1, **characterised in that** the suspension of step c) is left to stand for a period of between 15 and 120 minutes.

10. A process according to claim 1, **characterised in that** the suspension of step c) is left to stand for a period of between 20 and 60 minutes.

11. A process according to claim 1, **characterised in that** the temperature of step c) is between 15 and 30°C.

12. A process according to claim 1, **characterised in that** said organic solvent has a water content of less than or equal to 1% by weight.

13. A process according to claim 1, **characterised in that** said organic solvent is a polar organic solvent.

14. A process according to claim 13, **characterised in that** said polar organic solvent is selected from among acetone, tetrahydrofuran, C₂-C₈ ethers, C₂-C₈ acetates and mixtures thereof.

15. A process according to claim 13, **characterised in that** said polar organic solvent is acetone.

16. A process according to claim 1, **characterised in that** said organic solvent is a nonpolar organic solvent.

17. A process according to claim 16, **characterised in that** said nonpolar organic solvent is selected from among chloroform, methylene chloride, toluene and mixtures thereof.

18. A process according to claim 1, **characterised in that** it comprises an additional purification step by means of dissolution of the resultant tauroursodeoxycholic acid in deionised water and subsequent recrystallisation.

19. A process according to claim 18, **characterised in that** dissolution in deionised water is carried out at a temperature of less than 65°C.

20. A process according to claim 18, **characterised in that** dissolution in deionised water is carried out for a maximum period of 60 minutes.

21. A process according to claim 18, **characterised in that** the ratio by weight of tauroursodeoxycholic acid:deionised water is within the range from 0.1 to 1 relative to the dry weight of tauroursodeoxycholic acid.

22. A process according to claim 21, **characterised in that** the ratio by weight of tauroursodeoxycholic acid:deionised water is within the range from 0.4 to 0.6 relative to the dry weight of tauroursodeoxycholic acid.
